# EUROPÄISCHE PATENTSCHRIFT

(11) **EP 0 452 951 B1**
(45) Veröffentlichungstag und Bekanntmachung des Hinweises auf die Patenterteilung: **08.11.1995**
(21) Anmeldenummer: 91106326.1
(22) Anmeldetag: 19.04.1991
(51) Int. Cl.: A61F 13/15

(54) **Windelhöschen**
Diaper briefs
Couche-culotte

(30) Priorität: 19.04.1990 JP 42060/90
(43) Veröffentlichungstag der Anmeldung: 23.10.1991
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Nomura, Hironori, Iyomishima-shi, Ehime-ken (JP); Tanji, Hironori, Kawanoe-shi, Ehime-ken (JP); Takahashi, Tatsuo, Iyomishima-shi, Ehime-ken (JP); Nakano, Takumi, Kanonji-shi, Kagawa-ken (JP)
(74) Vertreter: Sperling, Rüdiger, Dipl.-Ing.

(56) Entgegenhaltungen:
- EP-A- 0 187 728
- FR-A- 2 345 955
- US-A- 4 909 804

## Beschreibung

Die vorliegende Erfindung betrifft ein Windelhöschen, das im wesentlichen eine obere Lage, eine untere Lage und einen zwischen diesen liegenden absorbierenden Kern umfaßt, und insbesondere ein solches Windelhöschen, das so ausgelegt ist, daß es mit umgeschlagenen seitlich gegenüberliegenden Seitenabschnitten gefaltet werden kann, so daß es sowohl von einem Hersteller auf einer Fertigungsstraße wirtschaftlich zu verpacken ist als auch von einem Verbraucher leicht zu handhaben ist.

Die wie vorstehend erwähnt eine obere Lage, eine untere Lage und einen zwischen diesen liegenden absorbierenden Kern umfassende Windel hat eine führende Marktposition in der Wegwerfwindelindustrie. Solche Windeln werden allgemein in offene Windeln und Höschenwindeln eingeteilt, wobei letztere in jüngerer Zeit aufgrund ihrer bequemen Benutzung die Aufmerksamkeit des Verbrauchers auf sich ziehen. Eine Windel dieser Art enthält oft um die Hüftöffnung und die Beinöffnungen angeordnete elastische Elemente, um diese Bereiche zu raffen. Manchmal wird als untere Lage dehnbares Material verwendet, um bei angelegter Windel eine gute Passform zu erreichen. Wirken auf eine derartige Windel keine Rückhaltekräfte, so neigt sie dazu, aufgrund der Kontraktion der entsprechenden Elemente und Materialien eine unförmige Gestalt anzunehmen. Zur Lösung dieses Problems gibt es eine Verpackungstechnik, die nach dem Stand der Technik an einer Fertigungsstraße verwendet wurde, bei der die einzelnen Produkte so flach wie möglich angeordnet werden, so daß mehrere Produkte auf einfache Weise übereinander stapelbar sind und anschließend in einem Beutel oder einem Karton verpackt werden können.

Bei den Windelhöschen mit einem zwischen einer oberen Lage und einer unteren Lage liegenden absorbierenden Kern war es schwierig, ein ansprechendes äußeres Erscheinungsbild der jeweiligen Packung zu erreichen, das den Käufern den Eindruck wertvoller Waren vermittelt, da diese Windeln aufgrund des Volumens des absorbierenden Kerns und der Kontraktion der um die Bein- und Hüftöffnungen angeordneten elastischen Elemente dazu neigen, eine unförmige Gestalt anzunehmen. Dieses Problem tritt in verstärktem Umfang auf, wenn die obere Lage und/oder die untere Lage aus dehnbarem Material bestehen. Um eine derartige Windel so flach wie möglich anzuordnen, waren für den Verpackungsvorgang an einer Fertigungsstraße nachteiligerweise nicht nur eine umfangreiche Ausrüstung, sondern auch ein erhöhter Arbeitsaufwand erforderlich.

Die flache Anordnung der Windelhöschen führte unvermeidlich zu einem komplizierten Übereinanderlegen von Vorder- und Hinterteil entlang der Hüftöffnung, wodurch es beim Anlegen der Windel nicht nur schwierig war, die Hüftöffnung aufzufinden, sondern auch das Trennen von Vorder- und Hinterteil der Windel voneinander, wobei der Rand der Hüftöffnung von den Fingern des Benutzers gehalten wurde, gewöhnlich sehr zeitaufwendig war.

### BESCHREIBUNG DER ERFINDUNG

Aus der US-PS 4 909 804 ist eine Windelhose zu Trainingszwecken für Kinder bekannt, bei der die Seitenabschnitte der mit ihren Außenseiten zu einer Windel zusammengelegten Vorform miteinander nach Art einer Verkettelung verbunden sind. Anschließend wird die Windel umgestülpt, so daß sich dann die Außenlage an der Außenseite befindet, wobei die miteinander verbundenen Seitenabschnitte nach innen gedreht sind.

Diese Windelhose ist in der Herstellung wegen des Kettelvorgangs wie auch wegen des zusätzlichen Arbeitsganges des Umstülpens aufwendig herzustellen und in der Handhabung und im Tragekomfort umständlich bzw. unbequem.

Der Erfindung liegt die Aufgabe zugrunde, eine Windelhose der genannten Art so zu verbessern, daß sie sowohl einfach herzustellen und auf einer Fertigungsstraße mit umgeschlagenen, seitlich gegenüberliegenden Seitenabschnitten so verpackt werden kann, daß sie eine ansprechende äußere Erscheinungsform aufweist und leicht zu handhaben ist.

Die vorstehende Aufgabe wird gelöst, indem erfindungsgemäß ein Windelhöschen mit wenigstens einer oberen Lage, einer unteren Lage und einem zwischen diesen liegenden absorbierenden Kern vorgesehen ist, wobei seitlich gegenüberliegende Seitenabschnitte des Windelhöschens, die sich von einer Hüftöffnung zu den entsprechenden Beinöffnungen erstrecken, auf eine in Längsrichtung verlaufende Mittellinie der Windel zu umgeschlagen gefaltet sind, so daß sie zwischen dem Vorderteil und dem Hinterteil der Windel angeordnet sind. Entsprechend einem weiteren Merkmal der Erfindung tritt die Umschlagfaltung der Seitenabschnitte entlang wenigstens einem Teil jedes Seitenrandes des absorbierenden Kerns auf.

Gemäß der vorliegenden Erfindung, bei der die seitlich gegenüberliegenden Seitenränder zwischen dem Vorderteil und dem Hinterteil entlang wenigstens einem Teil der jeweiligen Seitenkanten des voluminösen und steifen absorbierenden Kerns eingeschlagen gefaltet sind, erhält die auf diese Weise gefaltete Windel ihre äußere Form, die durch den relativ verformungsstabilen absorbierenden Kern bestimmt und stabilisiert wird. Eine derartige Anordnung erlaubt es auch, mehrere Windeln einfach zu stapeln und ordentlich in einem Beutel oder einem Karton zu verpacken, wodurch der Verpackungsablauf unter Verwendung einer relativ einfachen Verpackungsausrüstung schnell durchführbar ist. Darüber hinaus kann die Hüftöffnung einfach geöffnet werden, wenn die Windel angelegt werden soll, da der Vorderteil und der Hinterteil bereits vorher voneinander getrennt wurden.

Weitere Merkmale und Vorteile der Erfindung werden aus der folgenden, genaueren Beschreibung der bevorzugten Ausführungsformen in Verbindung mit den beigefügten Zeichnungen offenbar, die als Beispiele die Prinzipien der Erfindung erläutern.

### KURZE BESCHREIBUNG DER ZEICHNUNGEN

- Fig. 1: zeigt als Beispiel eine perspektivische Darstellung eines sogenannten Windelhöschens;
- Fig. 2: zeigt eine auseinandergezogene perspektivische Darstellung der in Fig. 1 gezeigten Windel;
- Fig. 3 u.4: zeigen eine perspektivische bzw. eine Schnittansicht einer Ausführungsform einer erfindungsgemäß aufgebauten Windel; und
- Fig. 5 u. 6: zeigen eine perspektivische bzw. eine Schnittansicht einer weiteren Ausführungsform einer erfindungsgemäß aufgebauten Windel.

### BEVORZUGTE AUSFÜHRUNGSFORMEN DER ERFINDUNG

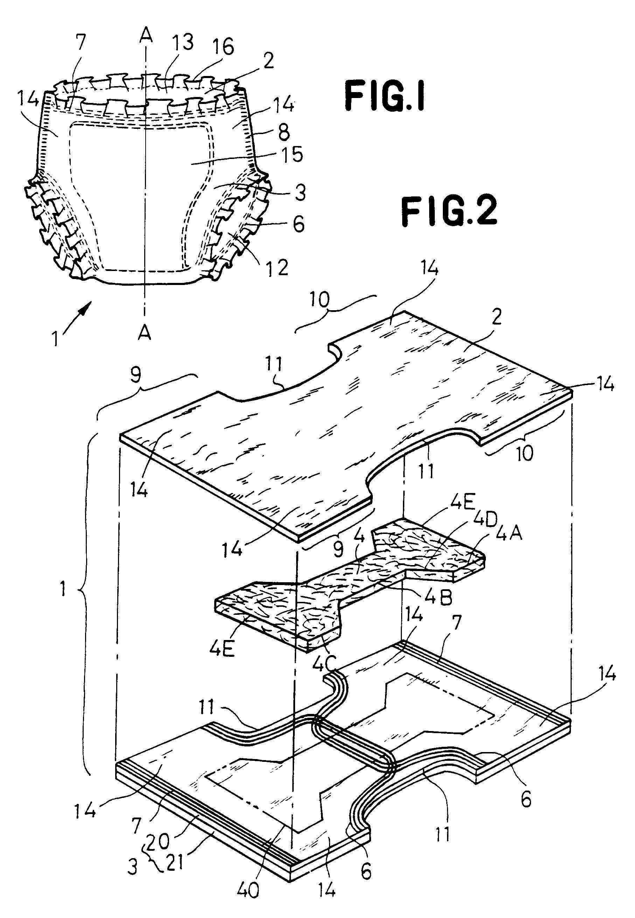

Fig. 1 zeigt ein sogenanntes Windelhöschen 1, das die Erfindung allgemein betrifft. Wie gezeigt umfaßt die Windel 1 eine obere Lage 2, eine untere Lage 3 und einen zwischen diesen liegenden absorbierenden Kern 4 (siehe Fig. 2). Weiter umfaßt die Windel 1 Gummifäden 6, die als elastische Elemente für jeweilige Beinöffnungen dienen, sowie Gummifäden 7, die als elastische Elemente für eine Hüftöffnung dienen. Die Seitenkanten von seitlich gegenüberliegenden Seitenabschnitten 14 sind jeweils durch Heißschmelzklebestreifen 8 verschlossen.

Fig. 2 ist eine auseinandergezogene perspektivische Darstellung, die schematisch die Anordnung der jeweiligen Einzelteile, die zur in Fig. 1 gezeigten Windel 1 zusammenzusetzen sind, zeigt, d. h. die obere Lage 2, die untere Lage 3, deren Größe im wesentlichen der der oberen Lage 2 entspricht, sowie den sanduhrförmigen Absorptionskern 4, wie er gewöhnlich für Wegwerfwindeln verwendet wird. Der Absorptionskern 4 ist mittels nicht dargestellten Heißschmelzklebers punktweise mit der Rückseite der oberen Lage 2 verklebt. Die untere Lage 3 ist so an der oberen Lage 2 angeordnet, daß sie den Absorptionskern 4 abdeckt. Die untere Lage 3 ist mittels nicht dargestellten Heißschmelzklebers an den Rändern ihres Umfangs mit der oberen Lage 2 verklebt. Die elastischen Elemente 6, 7 sind mittels nicht dargestellten Heißschmelzklebers in gedehntem Zustand punktweise auf die zur oberen Lage 2 weisende Fläche der unteren Lage 3 aufgeklebt. Nach dem Vollenden dieses Verklebens der elastischen Elemente 6, 7 wird die Windel entlang einer in der Länge der Windel im wesentlichen in deren Mitte liegenden Linie übereinandergefaltet, wobei Längsseitenbereiche 9, 10 übereinandergelegt werden. Diese so übereinandergelegten Längsseitenbereiche 9, 10 werden zur Bildung einer vollständigen Windel an den jeweiligen Heißschmelzklebestreifen 8 (siehe Fig. 1) heißversiegelt, worauf jeweils in der oberen und unteren Lage 2, 3 ausgebildete Ausschnitte 11 jeweilige Beinöffnungen 12 bilden (siehe Fig. 1) und die elastischen Elemente 6 um den äußeren Umfang der jeweiligen Beinlöcher 12 Raffungen bilden. Es versteht sich, daß die Bildung derartiger Raffungen vom Umfang der Erfindung gedeckt ist und daß daher hier keine weiteren Einzelheiten dazu beschrieben werden. Entsprechend bilden die an den in Längsrichtung gegenüberliegenden Enden der Windel angeordneten elastischen Elemente 7 zusammen eine Hüftöffnung 13 (siehe Fig. 1). Der Absorptionskern 4 hat ein sanduhrförmiges Profil mit in Längsrichtung verlaufenden Seitenrändern 4A, 4B, 4C, schräg verlaufenden Seitenrändern 4D und quer verlaufenden Rändern 4E.

Was die Werkstoffe der Windel 1 betrifft, kann die obere Lage 2 aus einem flüssigkeitsdurchlässigen, aus thermoplastischen Fasern aus Polyethylen, Polypropylen oder ähnlichem gebildeten Faservliesstoff bestehen und der Absorptionskern 4 kann aus lockerer Fasermasse bestehen, die 10 Gewichtsprozent hoch wasserabsorptives Polymerpulver enthält. Die untere Lage kann aus dehnbarer, wasserdichter Kunststoffolie 20 und dehnbarem Faservliesstoff 21 bestehen, der aus warmgekräuselten Polyethylen/Polypropylen-Zweikomponentenfasern gebildet ist, wobei beide Schichten mittels nicht dargestellten Heißschmelzklebers punktweise verklebt sind. Wie aus der auseinandergezogenen perspektivischen Darstellung in Fig. 2 ersichtlich ist, ist der Absorptionskern 4 so dimensioniert, daß er nur die jeweils mittleren Bereiche der oberen Lage 2 und der unteren Lage 3 einnimmt. Eine unterbrochene Linie 40 bezeichnet die Größe des Absorbtionskerns 4 in der Projektion auf die untere Lage 3.

In der wie vorstehend erläutert aufgebauten Windel 1 umfassen die seitlich gegenüberliegenden Seitenabschnitte 14, die sich von der Hüftöffnung 13 zu den jeweiligen Beinöffnungen 12 erstrecken, die obere und untere Lage 2, 3, nicht aber den Absorptionskern 4. In der Tat neigt die Windel 1 dazu, aufgrund nicht nur der Sperrigkeit des entlang der Mittellinie in der Länge der Windel 1 umgefalteten Absorptionskerns 4, sondern auch der Kontraktion der elastischen Elemente, der unteren Lage 3 und der weiteren elastischen Bauteile die Erscheinungsform einer unförmigen Masse anzunehmen. Unter Berücksichtigung dessen zeigt Fig. 1 die Windel 1 so gestreckt, daß die Anordnung der Windel 1 leicht verständlich ist.

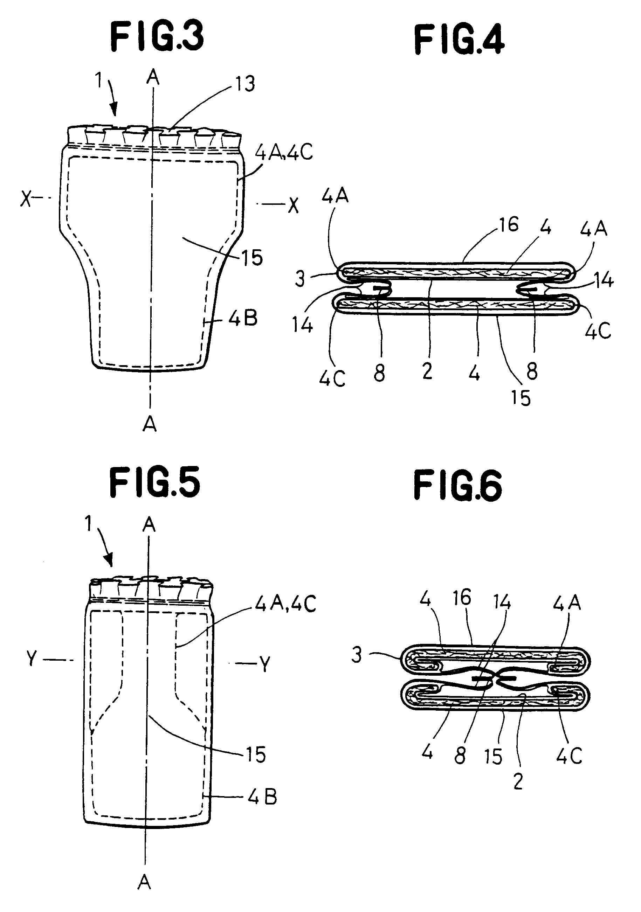

Fig. 3 und 4 zeigen eine gemäß der Erfindung aufgebaute Ausführungsform der Windel, die der in Fig. 1 gezeigten Windel 1 entspricht, wobei die seitlich gegenüberliegenden Seitenabschnitte 14, die sich von der Hüftöffnung 13 zu den jeweiligen Beinöffnungen 12 erstrecken, mit den entsprechenden Heißschmelzklebestreifen 8 voran auf eine in Längsrichtung verlaufende Mittellinie A-A zu umgeschlagen sind und zwischen einem Vorderteil 15 und einem Hinterteil 16 liegend angeordnet sind. Der Umriß der dergestalt gefalteten Windel folgt dem Profil des Absorptionskerns und umfaßt den ebenen Vorderteil 15 auf der Oberseite, den ebenen Hinterteil 16 auf der Unterseite und den dazwischenliegenden Absorptionskern 4. Somit ist die gesamte äußere Form der Windel durch den relativ steifen Absorptionskern bestimmt, womit mehrere einzelne Windeln leicht zu stapeln sind. Auf diese Weise ist es ebenso einfach, die Windeln in einer Fertigungsstraße in einen Beutel oder einen Karton zu packen. Darüber hinaus sind der Vorderteil 15 und der Hinterteil 16 durch die Stärke der umgeschlagenen Seitenabschnitte 14 voneinander getrennt, so daß die Anordnung der Hüftöffnung 13 hervorgehoben wird und ein Benutzer die Hüftöffnung 13 leicht öffnen kann, wobei der Rand am Umfang der Hüftöffnung 13 zwischen den Fingern des Benutzers gehalten wird.

Wenn die Windel 1 in dem in Fig. 1 gezeigten Zustand direkt zusammengedrückt wird, um ihr eine flachere Gestalt zu geben, und die beiden Seitenabschnitte 14 auf den Vorderteil 15 gefaltet werden, würden Vorder- und Hinterteil 15, 16 entlang der Hüftöffnung 13 in eine komplizierte Verbindung gebracht und die Anordnung der Hüftöffnung 13 würde in ungehöriger Weise gestört, so daß wesentlich mehr Zeit und Arbeitsaufwand zum Öffnen der Hüftöffnung 13 erforderlich wären, als es bei der erfindungsgemäß vorgesehenen Windel der Fall ist.

Unter Bezug auf Fig. 5 wird eine weitere bevorzugte Ausführungsform der Erfindung erläutert. Die Windel 1 ist in ähnlicher Weise wie die in Fig. 3 und 4 gezeigte Ausführungsform umgeschlagen gefaltet. Die seitlich gegenüberliegenden Seitenabschnitte 14 sind jedoch entlang den Seitenrandbereichen 4B des Absorptionskerns 4 umgeschlagen. Die beiden seitlich gegenüberliegenden Seitenrandbereiche 4B bilden den schmalsten Abschnitt des Absorptionskerns 4. Entsprechend sind die jeweils entlang den gegenüberliegenden quer verlaufenden Rändern 4E sich erstreckenden breiten Bereiche des sanduhrförmigen Absorptionskerns 4, d.h. die jeweils den Seitenrändern 4A, 4C benachbarten Bereiche, gemeinsam mit den seitlich gegenüberliegenden Seitenabschnitten 14 zwischen den Vorderteil 15 und den Hinterteil 16 umgeschlagen.

Fig. 6 ist eine entlang der Linie Y-Y in Fig. 5 verlaufende Schnittansicht, die die jeweils benachbart zu den Seitenrandbereichen 4A, 4C verlaufenden Bereiche des Absorptionskerns 4 zeigt, die gemeinsam mit den seitlich gegenüberliegenden Seitenabschnitten 14 umgeschlagen wurden.

Die die in Fig. 5 und 6 gezeigte Weise des Umschlagens erlaubt es, die Windel in einer in der in Fig. 5 gezeigten Ansicht im wesentlichen rechteckigen Form anzuordnen, erleichtert zusätzlich zu den durch die Ausführungsform 1 erzielten Vorteile das Verpacken der Windel und verbessert das äußere Erscheinungsbild.

## Patentansprüche

1. Windelhöschen mit einen Vorderteil (15) und einem Hinterteil (16) sowie beide Teile miteinander verbindenden Seitenabschnitte (14,14), bestehend aus einem Absorbtionskern und wenigstens einer oberen flüssigkeitsdurchlässigen und einer unteren flüssigkeitsundurchlässigen Lage (2;3) sanduhrförmiger Gestalt, die den Absorbtionskern (4) sandwichartig zwischen sich einschließen und und deren Seitenränder seitlich über den Absorbtionskern (4) hinausragen, wobei die sich gegenüberliegenden Seitenabschnitte der Windel, die sich von einer Hüftöffnung (13) zu jeweiligen Beinöffnungen (12) erstrecken, von aufeinandergelegten und miteinander verbundenen Längsseitenbereichen (9,9;1o,1o) des Vorderteils (15) bzw Hinterteils (16) gebildet sind, dadurch **gekennzeichnet,**
daß die Längsseitenbereiche (9,9;10,10) jeweils mit ihrer flüssigkeitdurchlässigen Lage (2,2) aufeinandergelegt miteinander verbunden und die Seitenabschnitte (14) auf eine in Längsrichtung verlaufende Mittellinie der Windel zu zumindest bis zum Rand des Absorbtionskerns eingeschlagen sind, so daß sie zwischen dem Vorderteil (15) und dem Hinterteil (16) der Windel angeordnet sind.

2. Windel nach Anspruch 1, wobei die Einschlagfaltlinie zumindest teilweise durch einem Bereich jeder Seite des Absorptionskerns (4) verläuft.

## Claims

1. Nappy pants with a front portion (15), a back portion (16) and side sections (14, 14) connecting both portions together, comprising an absorbent core and at least one upper liquid-permeable and one lower liquid-impermeable layer (2; 3) of hourglass shape, which enclose the absorbent core (4) between them in the manner of a sandwich, and whose lateral edges project laterally over the absorbent core (4), the oppositely located side sections of the nappy, which extend from a hip opening (13) to the respective leg openings (12), being formed by longitudinal side areas (9, 9; 10, 10) of the front portion (15) or back portion (16) which are superimposed one on the other and interconnected,
characterised in that the longitudinal side areas (9, 9; 10, 10) with their respective liquid-permeable layer (2, 2) are laid one upon the other and interconnected, and the side sections (14) are inserted on a centre line of the nappy extending in the longitudinal direction at least as far as the edge of the absorbent core, so that they are positioned between the front portion (15) and the back portion (16) of the nappy.

2. Nappy according to claim 1, in which the insertion fold line extends at least partly through an area of each side of the absorbent core (4).

## Revendications

1. Couche-culotte comportant une partie avant (15) et une partie arrière (16) et des portions latérales (14, 14) reliant ces deux parties, et se composant d'un corps central absorbant et d'au moins une couche supérieure perméable aux liquides et d'une couche inférieure imperméable aux liquides (2 ; 3) en forme de sablier, qui prennent le corps central absorbant (4) en sandwich et dont les bords latéraux dépassent latéralement du corps central absorbant (4), couche-culotte dans laquelle les portions latérales opposées du lange, qui s'étendent d'une ouverture de passage des hanches (13) jusqu'aux ouvertures de passage des jambes (12) respectives, sont constituées par des zones latérales longitudinales (9, 9 ; 10, 10) de la partie avant (15) et de la partie arrière (16) posées l'une sur l'autre et reliées entre elles, caractérisée en ce que les zones latérales longitudinales (9, 9 ; 10, 10) sont jointes avec leurs couches perméables aux liquides (2, 2) posées l'une sur l'autre, et en ce que les portions latérales (14) sont rabattues intérieurement sur une ligne médiane orientée dans le sens de la longueur du lange, au moins jusqu'au bord du corps central absorbant, de sorte qu'elles sont disposées entre la partie avant (15) et la partie arrière (16) du lange.

2. Lange selon la revendication 1, dans lequel la ligne de pliage s'étend au moins en partie au-travers d'une zone de chaque côté du corps central absorbant (4).
